# FASCICULE DE BREVET EUROPEEN

(11) **EP 0 998 259 B1**
(45) Date de publication et mention de la délivrance du brevet: **19.05.2004**
(21) Numéro de dépôt: 98946515.8
(22) Date de dépôt: 28.09.1998
(51) Int. Cl.: A61K 7/13

(54) **COMPOSITION DE TEINTURE D'OXYDATION DES FIBRES KERATINIQUES**
ZUSAMMENSETZUNG FÜR DIE OXYDATIONSFÄRBUNG VON KERATINISCHEN FASERN
OXIDATION DYEING COMPOSITION FOR KERATIN FIBRES

(30) Priorité: 03.10.1997 FR 9712351
(43) Date de publication de la demande: 10.05.2000
(73) Titulaire: L'OREAL, 75008 Paris (FR)
(72) Inventeur: DE LA METTRIE, Roland, F-78110 Le Vésinet (FR); COTTERET, Jean, F-78480 Verneuil-sur-Seine (FR); DE LABBEY, Arnaud, F-93600 Aulnay sous Bois (FR); MAUBRU, Mireille, F-78400 Chatou (FR)
(74) Mandataire: Goulard, Sophie
(86) Numéro de dépôt international: PCT/FR1998/002074
(87) Numéro de publication internationale: WO 1999/017729

(56) Documents cités:
- EP-A- 0 716 846
- EP-A- 0 795 313
- US-A- 4 961 925

## Description

L'invention a pour objet une composition pour la teinture d'oxydation des fibres kératiniques, et en particulier des fibres kératiniques humaines telles que les cheveux comprenant, dans un milieu approprié pour la teinture, au moins une première base d'oxydation choisie parmi les dérivés de paraphénylènediamine, les bases doubles, les orthoaminophénols et les bases hétérocycliques, au moins une seconde base d'oxydation choisie parmi les para-aminophénols, au moins un méta-aminophénol à titre de coupleur, et au moins une enzyme de type oxydo-réductase à 2 électrons en présence d'au moins un donneur pour ladite enzyme, ainsi que le procédé de teinture mettant en oeuvre cette composition.

Il est connu de teindre les fibres kératiniques et en particulier les cheveux humains avec des compositions tinctoriales conienant des précurseurs de colorant d'oxydation, en particulier des ortho ou paraphénylènediamines, des ortho ou paraaminophénols, des bases hétérocycliques, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

La coloration d'oxydation des fibres kératiniques est généralement réalisée en milieu alcalin, en présence de peroxyde d'hydrogène. Toutefois, l'utilisation des milieux alcalins en présence de peroxyde d'hydrogène présentent pour inconvénient d'entraîner une dégradation non négligeable des fibres, ainsi qu'une décoloration importante des fibres kératiniques qui n'est pas toujours souhaitable.

La coloration d'oxydation des fibres kératiniques peut également être réalisée à l'aide de systèmes oxydants différents du peroxyde d'hydrogène tels que des systèmes enzymatiques. Ainsi il a déjà été proposé de teindre les fibres kératiniques, notamment dans la demande de brevet EP-A-0 310 675, avec des compositions comprenant une base d'oxydation et éventuellement un coupleur, en association avec des enzymes telles que la pyranose-oxydase, la glucose-oxydase ou bien l'uricase, en présence d'un donneur pour lesdites enzymes.

Dans la demande de brevet EP 0 795 313, il a été proposé des compositions pour la teinture des fibres kératiniques comprenant du 4-amino-3-méthyl phénol, du 5-amino-2-méthyl phénol, de la glucose oxydase et du D-glucose.

Dans la demande de brevet EP 0 716 846, il a été proposé des compositions pour la teinture des fibres kératiniques comprenant de la paraphénylènediamine, du para-amino phénol, du méta-aminophénol, de l'uricase et un donneur pour ladite uricase.

Ces procédés de teinture, bien qu'étant mis en oeuvre dans des conditions n'entraînant pas une dégradation des fibres kératiniques comparable à celle engendrée par les teintures réalisées en présence de peroxyde d'hydrogène, conduisent à des colorations ne donnant pas entière satisfaction notamment du point de vue de leur intensité et de leur résistance vis à vis des diverses agressions que peuvent subir les cheveux.

Or, la demanderesse vient maintenant de découvrir qu'il est possible d'obtenir de nouvelles teintures, capables de conduire à des colorations puissantes sans engendrer de dégradation significative des fibres kératiniques, peu sélectives et résistant bien aux diverses agressions que peuvent subir les fibres, en associant au moins première base d'oxydation choisie parmi les dérivés de paraphénylènediamine différents de la paraphénylènediamine , les bases doubles, les orthoaminophénols et les bases hétérocycliques, au moins une seconde base d'oxydation choisie parmi les para-aminophénols, au moins un méta-aminophénol à titre de coupleur, et au moins une enzyme de type oxydo-réductase à 2 électrons en présence d'au moins un donneur pour ladite enzyme.

Cette découverte est à la base de la présente invention.

L'invention a donc pour premier objet une composition prête à l'emploi, pour la teinture d'oxydation des fibres kératiniques et en particulier des fibres kératiniques humaines telles que les cheveux, caractérisée par le fait qu'elle comprend, dans un milieu approprié pour la teinture :
- au moins une première base d'oxydation choisie parmi les dérivés de paraphénylènediamine différents de la paraphénylènediamine, les bases doubles, les orthoaminophénols et les bases hétérocycliques,
- au moins une seconde base d'oxydation choisie parmi les paraaminophénols,
- au moins un méta-aminophénol à titre de coupleur,
- au moins une enzyme de type oxydo-réductase à 2 électrons,
- et au moins un donneur pour ladite enzyme.

La composition tinctoriale prête à l'emploi conforme à l'invention conduit à des colorations puissantes présentant une faible sélectivité et d'excellentes propriétés de résistances à la fois vis à vis des agents atmosphériques tels que la lumière et les intempéries et vis à vis de la transpiration et des différents traitements que peuvent subir les cheveux (lavages, déformations permanentes).

L'invention a également pour objet un procédé de teinture d'oxydation des fibres kératiniques mettant en oeuvre cette composition tinctoriale prête à l'emploi.

La ou les oxydo-réductases à 2 électrons utilisées dans la composition tinctoriale prête à l'emploi conforme à l'invention peuvent notamment être choisies parmi les pyranose oxydases, les glucose oxydases, les glycérol oxydases, les lactates oxydases, les pyruvate oxydases, et les uricases.

Selon l'invention, l'oxydo-réductase à 2 électrons est de préférence choisie parmi les uricases d'origine animale, microbiologique ou biotechnologique.

A titre d'exemple, on peut notamment citer l'uricase extraite de foie de sanglier, l'uricase d'Arthrobacter globiformis, ainsi que l'uricase d'Aspergillus flavus.

La ou les oxydo-réductases à 2 électrons peuvent être utilisées sous forme cristalline pure ou sous une forme diluée dans un diluant inerte pour ladite oxydo-réductase à 2 électrons.

La ou les oxydo-réductases à 2 électrons conformes à l'invention représentent de préférence de 0,01 à 20 % en poids environ du poids total de la composition tinctoriale prête à l'emploi, et encore plus préférentiellement de 0,1 à 5 % en poids environ de ce poids.

Selon l'invention, on entend par donneur, les différents substrats participant au fonctionnement de ladite ou desdites oxydo-réductases à 2 électrons.

La nature du donneur (ou substrat) pour ladite enzyme varie en fonction de la nature de l'oxydo-réductase à 2 électrons qui est utilisée. Par exemple, à titre de donneur pour les pyranose oxydases, on peut citer le D-glucose, le L-sorbose et le D-xylose ; à titre de donneur pour les glucose oxydases, on peut citer le D-glucose, à titre de donneur pour les glycérol oxydases, on peut citer le glycérol et la dihydroxyacétone ; à titre de donneur pour les lactate oxydases, on peut citer l'acide lactique et ses sels ; à titre de donneur pour les pyruvate oxydases, on peut citer l'acide pyruvique et ses sels ; et enfin à titre de donneur pour les uricases, on peut citer l'acide urique et ses sels.

Le ou les donneurs (ou substrats) utilisés conformément à l'invention représentent de préférence de 0,01 à 20 % en poids environ du poids total de la composition tinctoriale prête à l'emploi conforme à l'invention et encore plus préférentiellement de 0,1 à 5 % en environ de ce poids.

Parmi les para-aminophénols utilisables à titre de deuxième base d'oxydation dans les compositions tinctoriales conformes à l'invention, on peut notamment citer les composés répondant à la formule (II) suivante, et leurs sels d'addition avec un acide : 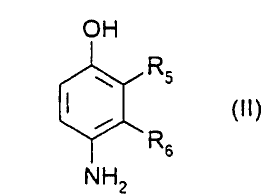 dans laquelle :
- R₅ représente un atome d'hydrogène ou d'halogène, un radical alkyle en C₁-C₄, monohydroxyalkyle en C₁-C₄, alcoxy(C₁-C₄)alkyle(C₁-C₄), aminoalkyle en C₁-C₄ ou hydroxyalkyl(C₁-C₄)aminoalkyle en C₁-C₄,
- R₆ représente un atome d'hydrogène ou d'halogène, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄),
étant entendu qu'au moins un des radicaux R₅ ou R₆ représente un atome d'hydrogène.

Parmi les para-aminophénols de formule (II) ci-dessus, on peut plus particulièrement citer le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Le ou les para-aminophénols utilisables à titre de seconde base d'oxydation représentent de préférence de 0,0005 à 12 % en poids environ du poids total de la composition tinctoriale prête à l'emploi conforme à l'invention, et encore plus préférentiellement de 0,005 à 6 % en poids environ de ce poids.

Le ou les méta-aminophénols pouvant être utilisés à titre coupleur dans la composition tinctoriale prête à l'emploi conforme à l'invention sont de préférence choisis parmi les composés de formule (III) suivante, et leurs sels d'addition avec un acide : 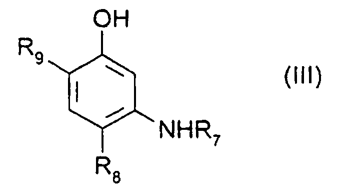 dans laquelle :
- R₇ représente un atome d'hydrogène, un radical alkyle en C₁-C₄, monohydroxyalkyle en C₁-C₄ ou polyhydroxyalkyle en C₂-C₄,
- R₈ représente un atome d'hydrogène, un radical alkyle en C₁-C₄, alcoxy en C₁-C₄ ou un atome d'halogène choisi parmi le chlore, le brome ou le fluor,
- R₉ représente un atome d'hydrogène, un radical alkyle en C₁-C₄, alcoxy en C₁-C₄, monohydroxyalkyle en C₁-C₄, polyhydroxyalkyle en C₂-C₄, monohydroxyalcoxy en C₁-C₄ ou polyhydroxyalcoxy en C₂-C₄.

Parmi les méta-aminophénols de formule (III) ci-dessus, on peut plus particulièrement citer le méta-aminophénol, le 5-amino 2-méthoxy phénol, le 5-amino 2-(β-hydroxyéthyloxy) phénol, le 5-amino 2-méthyl phénol, le 5-N-(β-hydroxyéthyl)amino 2-méthyl phénol, le 5-N-(β-hydroxyéthyl)amino 4-méthoxy 2-méthyl phénol, le 5-amino 4-méthoxy 2-méthyl phénol, le 5-amino 4-chloro 2-méthyl phénol, le 5-amino 2,4-diméthoxy phénol, le 5-(γ-hydroxypropylamino) 2-méthyl phénol, et leurs sels d'addition avec un acide.

Le ou les méta-aminophénols utilisables à titre de coupleur représentent de préférence de 0,0001 à 8 % en poids environ du poids total de la composition tinctoriale prête à l'emploi, et encore plus préférentiellement de 0,005 à 5 % en poids environ de ce poids.

Parmi les dérivés de paraphénylènediamine utilisables à titre première base d'oxydation dans la composition tinctoriale prête à l'emploi conforme à l'invention, on peut notamment citer les composés de formule (I) suivante, et leurs sels d'addition avec un acide : 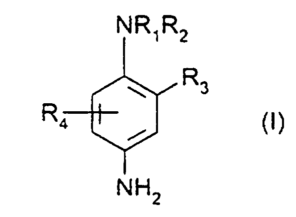 dans laquelle :
- R₁ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄), alkyle en C₁-C₄ substitué par un groupement azoté, phényle ou 4'-aminophényle ;
- R₂ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄) ou alkyle en C₁-C₄ substitué par un groupement azoté ;
- R₃ représente un atome d'hydrogène, un atome d'halogène tel qu'un atome de chlore, de brome, d'iode ou de fluor, un radical alkyle en C₁-C₄, monohydroxyalkyle en C₁-C₄, hydroxyalcoxy en C₁-C₄, acétylaminoalcoxy en C₁-C₄, mésylaminoalcoxy en C₁-C₄ ou carbamoylaminoalcoxy en C₁-C₄,
- R₄ représente un atome d'hydrogène, d'halogène ou un radical alkyle en C₁-C₄ ;
étant entendu qu'au moins un des radicaux R₁ à R₄ est différent d'un atome d'hydrogène.

Parmi les groupements azotés de la formule (I) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les dérivés de paraphénylènediamine de formule (I) ci-dessus, on peut plus particulièrement citer la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxy-éthyl)amino 2-méthyl aniline, la 4-N,N-bis-(β-hydroxy-éthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.

Parmi les dérivés de paraphénylènediamine de formule (I) ci-dessus, on préfère tout particulièrement la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphényiènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide.

Selon l'invention, on entend par bases doubles, les composés comportant au moins deux noyaux aromatiques sur lesquels sont portés des groupements amino et/ou hydroxyle.

Parmi les bases doubles utilisables à titre de première base d'oxydation dans la composition tinctoriale prête à l'emploi conforme à l'invention, on peut notamment citer les composés de formule (IV) suivante, et leurs sels d'addition avec un acide : 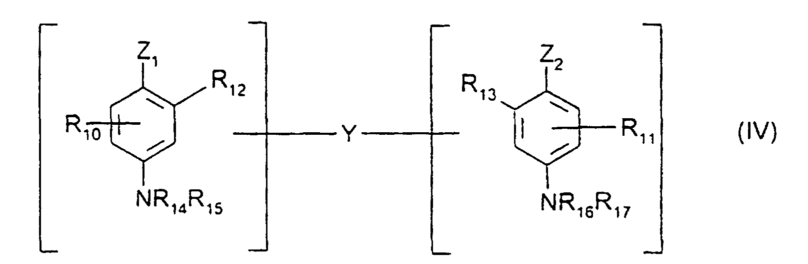 dans laquelle :
- Z₁ et Z₂, identiques ou différents, représentent un radical hydroxyle ou -NH₂ pouvant être substitué par un radical alkyle en C₁-C₄ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et eventuellement substituée par un ou plusieurs radicaux hydroxyle ou alcoxy en C₁-C₆ ;
- R₁₀ et R₁₁ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
- R₁₂, R₁₃, R₁₄, R₁₅, R₁₆ et R₁₇, identiques ou différents, représentent un atome d'hydrogène, un bras de liaison Y ou un radical alkyle en C₁-C₄ ;
étant entendu que les composés de formule (IV) ne comportent qu'un seul bras de liaison Y par molécule.

Parmi les groupements azotés de la formule (IV) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les bases doubles de formule (IV) ci-dessus, on peut plus particulièrement citer le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

Parmi ces bases doubles de formule (IV), le N,N'-bis-(β-hydroxyéthyl) N,N'-bis-(4'-aminophényl) 1,3-diamino propanol, le 1,8-bis-(2,5-diaminophénoxy)-3,5-dioxaoctane ou l'un de leurs sels d'addition avec un acide sont particulièrement préférés.

Parmi les orthoaminophénols utilisables à titre de première base d'oxydation dans la composition tinctoriale prête à l'emploi conforme à l'invention, on plus particulièrement citer le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques utilisables à titre de première base d'oxydation dans la composition tinctoriale conforme à l'invention, on peut plus particulièrement citer les dérivés pyridiniques, les dérivés pyrimidiniques, les dérivés pyrazoliques, les dérivés pyrazolo-pyrimidiniques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demandes de brevet WO 96/15765, comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

Parmi les dérivés pyrazolo-pyrimidiniques, on peut plus particulièrement citer les pyrazolo-[1,5-a]-pyrimidines de formule (V) suivante, leurs sels d'addition avec un acide ou avec une base et leurs formes tautomères, lorsqu'il existe un équilibre tautomérique : 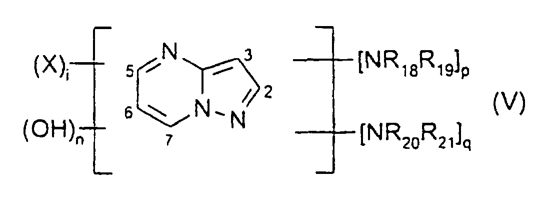 dans laquelle :
- R₁₈, R₁₉, R₂₀ et R₂₁, identiques ou différents désignent, un atome d'hydrogène, un radical alkyle en C₁-C₄, un radical aryle, un radical hydroxyalkyle en C₁-C₄, un radical polyhydroxyalkyle en C₂-C₄, un radical (C₁-C₄)alcoxy alkyle en C₁-C₄, un radical aminoalkyle en C₁-C₄ (l'amine pouvant être protégée par un radical acétyle, uréido ou sulfonyle), un radical (C₁-C₄)alkylamino alkyle en C₁-C₄, un radical di-[(C₁-C₄)alkyl] amino alkyle en C₁-C₄ (les radicaux dialkyles pouvant former un cycle carboné ou un hétérocycle à 5 ou 6 chaînons), un radical hydroxy(C₁-C₄)alkyl- ou di-[hydroxy(C₁-C₄)alkyl]-amino alkyle en C₁-C₄ ;
- les radicaux X désignent , identiques ou différents, un atome d'hydrogène, un radical alkyle en C₁-C₄, un radical aryle, un radical hydroxyalkyle en C₁-C₄, un radical polyhydroxyalkyle en C₂-C₄, un radical amino alkyle en C₁-C₄, un radical (C₁-C₄)alkyl amino alkyle en C₁-C₄, un radical di-[(C₁-C₄)alkyl] amino alkyle en C₁-C₄ (les dialkyles pouvant former un cycle carboné ou un hétérocycle à 5 ou 6 chaînons), un radical hydroxy(C₁-C4)alkyl ou di-[hydroxy(C₁-C₄ )alkyl]amino alkyle en C₁-C₄, un radical amino, un radical (C₁-C₄)alkyl- ou di-[(C₁-C₄)alkyl]-amino ; un atome d'halogène, un groupe acide carboxylique, un groupe acide sulfonique ;
- i vaut 0, 1, 2 ou 3 ;
- p vaut 0 ou 1 ;
- q vaut 0 ou 1 ;
- n vaut 0 ou 1 ;
sous réserve que :
- la somme p + q est différente de 0 ;
- lorsque p + q est égal à 2, alors n vaut 0 et les groupes NR₁₈R₁₉ et NR₂₀R₂₁ occupent les positions (2,3) ; (5,6) ; (6,7) ; (3,5) ou (3,7) ;
- lorsque p + q est égal à 1 alors n vaut 1 et le groupe NR₁₈R₁₉ (ou NR₂₀R₂₁) et le groupe OH occupent les positions (2,3) ; (5,6) ; (6,7) ; (3,5) ou (3,7) ;

Lorsque les pyrazolo-[1,5-a]-pyrimidines de formule (V) ci-dessus sont telles qu'elles comportent un groupe hydroxyle sur l'une des positions 2, 5 ou 7 en α d'un atome d'azote, il existe un équilibre tautomérique représenté par exemple par le schéma suivant :

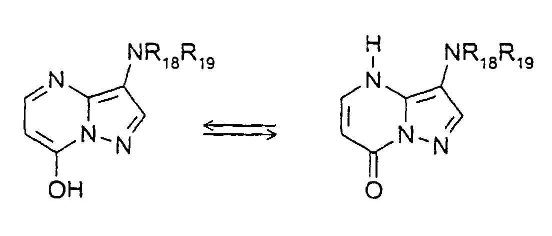

Parmi les pyrazolo-[1,5-a]-pyrimidines de formule (V) ci-dessus on peut notamment citer :
- la pyrazolo-[1,5-a]-pyrimidine-3,7-diamine ;
- la 2,5-diméthyl pyrazolo-[1,5-a]-pyrimidine-3,7-diamine ;
- la pyrazolo-[1,5-a]-pyrimidine-3,5-diamine ;
- la 2,7-diméthyl pyrazolo-[1,5-a]-pyrimidine-3,5-diamine ;
- le 3-amino pyrazolo-[1,5-a]-pyrimidin-7-ol
- le 3-amino pyrazolo-[1,5-a]-pyrimidin-5-ol
- le 2-(3-amino pyrazolo-[1,5-a]-pyrimidin-7-ylamino)-éthanol
- le 2-(7-amino pyrazolo-[1,5-a]-pyrimidin-3-ylamino)-éthanol
- le 2-[(3-Amino-pyrazolo[1,5-a]pyrimidin-7-yl)-(2-hydroxy-éthyl)-amino]-éthanol
- le 2-[(7-Amino-pyrazolo[1,5-a]pyrimidin-3-yl)-(2-hydroxy-éthyl)-amino]-éthanol
- la 5,6-diméthyl pyrazolo-[1,5-a]-pyrimidine-3,7-diamine ;
- la 2,6-diméthyl pyrazolo-[1,5-a]-pyrimidine-3,7-diamine ;
- la 2, 5, N 7, N 7-tetraméthyl pyrazolo-[1,5-a]-pyrimidine-3,7-diamine ;
et leurs sels d'addition et leurs formes tautomères, lorsqu'il existe un équilibre tautomérique.

Les pyrazolo-[1,5-a]-pyrimidines de formule (V) ci-dessus peuvent être préparées par cyclisation à partir d'un aminopyrazole selon les synthèses décrites dans les références suivantes :
- EP 628559 BEIERSDORF-LILLY
- R. Vishdu, H. Navedul, Indian J. Chem., 34b (6), 514, 1995.
- N.S. Ibrahim, K.U. Sadek, F.A. Abdel-Al, Arch. Pharm., 320, 240, 1987.
- R.H. Springer, M.B. Scholten, D.E. O'Brien, T. Novinson, J.P. Miller, R.K. Robins, J. Med. Chem., 25, 235, 1982.
- T. Novinson, R.K. Robins, T.R. Matthews, J. Med. Chem., 20, 296, 1977.
- US 3907799 ICN PHARMACEUTICALS

Les pyrazolo-[1,5-a]-pyrimidines de formule (V) ci-dessus peuvent également être préparées par cyclisation à partir d'hydrazine selon les synthèses décrites dans les références suivantes :
- A. McKillop et R.J. Kobilecki, Heterocycles, 6(9), 1355, 1977.
- E. Alcade, J. De Mendoza, J.M. Marcia-Marquina, C. Almera, J. Elguero, J. Heterocyclic Chem., 11(3), 423, 1974.
- K. Saito, I. Hori, M. Higarashi, H. Midorikawa, Bull. Chem. Soc. Japan, 47(2), 476, 1974.

Le ou les dérivés de paraphénylènediamine et/ou la ou les bases doubles et/ou les ou les orthoaminophénols et/ou la ou les bases hétérocycliques utilisables à titre de première base d'oxydation représentent de préférence de 0,0005 à 12 % en poids environ du poids total de la composition tinctoriale prête à l'emploi conforme à l'invention, et encore plus préférentiellement de 0,005 à 6 % en poids environ de ce poids.

La composition tinctoriale prête à l'emploi conforme à l'invention peut en outre contenir un ou plusieurs coupleurs additionnels différents des métaaminophénols utilisés selon l'invention et/ou un ou plusieurs colorants directs notamment pour modifier les nuances ou les enrichir en reflets.

Parmi les coupleurs pouvant être présents à titre additionnel dans la composition tinctoriale prête à l'emploi conforme à l'invention, on peut notamment citer les méta-phénylènediamines, les métadiphénols, les coupleurs hétérocycliques, et leurs sels d'addition avec un acide.

Lorsqu'ils sont présents ces coupleurs additionnels représentent de préférence de 0,0001 à 10 % en poids environ du poids total de la composition tinctoriale prête à l'emploi et encore plus préférentiellement de 0,005 à 5 % en poids environ de ce poids.

D'une manière générale, les sels d'addition avec un acide utilisables dans le cadre des compositions tinctoriales de l'invention (bases d'oxydation et coupleurs) sont notamment choisis parmi les chlorhydrates, les bromhydrates, les sulfates et les tartrates, les lactates et les acétates.

Le milieu approprié pour la teinture (ou support) de la composition tinctoriale prête à l'emploi conforme à l'invention est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols en C₁-C₄, tels que l'éthanol et l'isopropanol ; le glycérol ; les glycols et éthers de glycols comme le 2-butoxyéthanol, le propyléneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale prête à l'emploi, et encore plus préférentiellement entre 5 et 30 % en poids environ.

Le pH de la composition prête à l'emploi conforme à l'invention est choisi de telle manière que l'activité enzymatique de l'oxydo-réductase à 2 électrons soit suffisante. Il est généralement compris entre 5 et 11 environ, et de préférence entre 6,5 et 10 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines, le 2-méthyl 2-amino propanol ainsi que leurs dérivés, les hydroxydes de sodium ou de potassium et les composés de formule (VI) suivante : 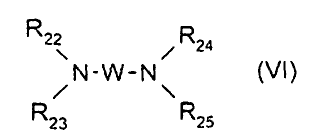 dans laquelle W est un reste propylène éventuellement substitué par un groupement hydroxyle ou un radical alkyle en C₁-C₄ ; R₂₂, R₂₃, R₂₄ et R₂₅, identiques ou différents, représentent un atome d'hydrogène, un radical alkyle en C₁-C₄ ou hydroxyalkyle en C₁-C₄.

La composition tinctoriale prête à l'emploi conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des enzymes différentes des oxydo-réductases à 2 électrons utilisées conformément à l'invention telles que par exemples des peroxydases, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Bien entendu, l'homme de l'art veillera à choisir ce ou ces éventuels composés complémentaires de manière telle que les propriétés avantageuses attachées intrinsèquement à la composition tinctoriale prête à l'emploi conforme à l'invention ne soient pas, ou substantiellement pas, altérées par la ou les adjonctions envisagées.

La composition tinctoriale prête à l'emploi conforme à l'invention peut se présenter sous des formes diverses, telles que sous forme de liquides, de crèmes, de gels, éventuellement pressurisés, ou sous toute autre forme appropriée pour réaliser une teinture des fibres kératiniques, et notamment des cheveux humains. Dans ce cas, les colorants d'oxydation et la ou les oxydo-réductases à 2 électrons sont présents au sein de la même composition prête à l'emploi, et par conséquent ladite composition doit être exempte d'oxygène gazeux, de manière à éviter toute oxydation prématurée du ou des colorants d'oxydation.

L'invention a également pour objet un procédé de teinture des fibres kératiniques et en particulier des fibres kératiniques humaines telles que les cheveux mettant en oeuvre la composition tinctoriale prête à l'emploi telle que définie précédemment.

Selon ce procédé, on applique sur les fibres au moins une composition tinctoriale prête à l'emploi telle que définie précédemment, pendant un temps suffisant pour développer la coloration désirée, après quoi on rince, on lave éventuellement au shampooing, on rince à nouveau et on sèche.

Le temps nécessaire au développement de la coloration sur les fibres kératiniques est généralement compris entre 3 et 60 minutes et encore plus précisément 5 et 40 minutes.

Selon une forme de réalisation particulière de l'invention, le procédé comporte une étape préliminaire consistant à stocker sous forme séparée, d'une part, une composition (A) comprenant, dans un milieu approprié pour la teinture, au moins une première base d'oxydation choisie parmi les dérivés de paraphénylènediamine, les bases doubles, les orthoaminophénols et les bases hétérocycliques, au moins une seconde base d'oxydation choisie parmi les para-aminophénols, au moins un méta-aminophénol à titre de coupleur et, d'autre part, une composition (B) renfermant, dans un milieu approprié pour la teinture, au moins une enzyme de type oxydo-réductase à 2 électrons en présence d'au moins un donneur pour ladite enzyme, puis à procéder à leur mélange au moment de l'emploi avant d'appliquer ce mélange sur les fibres kératiniques.

Un autre objet de l'invention est un dispositif à plusieurs compartiments ou "kit" de teinture ou tout autre système de conditionnement à plusieurs compartiments dont un premier compartiment renferme la composition (A) telle que définie ci-dessus et un second compartiment renferme la composition (B) telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant en limiter la portée.

### EXEMPLES

### EXEMPLES 1 et 2 DE TEINTURE

On a préparé les compositions tinctoriales prêtes à l'emploi suivantes (teneurs en grammes) :

| **COMPOSITION** | **1** | **2** |
|---|---|---|
| Dichlorhydrate de 2-β-hydroxyéthyl paraphénylènediamine (base d'oxydation) | 0,45 | 0,45 |
| Para-aminophénol (base d'oxydation) | 0,1 | 0,1 |
| Méta-aminophénol (coupleur) | 0,1 | - |
| 2-méthyl 5-amino phénol (coupleur) | - | 0,13 |
| Uricase d'Arthrobacter globiformis à 20 Unités Internationales (U.I.) / mg, commercialisée par la société Sigma | 1,5 | 1,5 |
| Acide urique | 1,5 | 1,5 |
| Support de teinture commun (*) | (*) | (*) |
| Eau déminéralisée q.s.p. | 100 g | 100 g |
| (*) : Support de teinture commun : - Ethanol 20,0 g - Hydroxyéthylcellulose vendue sous la dénomination NATROSOL 250 HR ® par la société AQUALON 1,0 g - Alkyl (C₈-C₁₀) polyglucoside en solution aqueuse à 60 % de matière active (M.A.) tamponné par du citrate d'ammonium (0,5%), vendu sous la dénomination ORAMIX CG110 ® par la société SEPPIC 8,0 g - Monoéthanolamine q.s. pH = 9,5 | | |

Chacune des compositions tinctoriales prêtes à l'emploi décrites ci-dessus a été appliquée sur des mèches de cheveux gris naturels à 90 % de blancs pendant 30 minutes. Les cheveux ont ensuite été rincés, lavés avec un shampooing standard, puis séchés.

Les cheveux ont été teints dans les nuances figurant dans le tableau ci-après :

| **EXEMPLE** | **Nuance obtenue** |
|---|---|
| **1** | Blond foncé nacré |
| **2** | Blond foncé acajou |

## Revendications

1. Composition prête à l'emploi, pour la teinture d'oxydation des fibres kératiniques et en particulier des fibres kératiniques humaines telles que les cheveux, **caractérisée par le fait qu'**elle comprend, dans un milieu approprié pour la teinture :
- au moins une première base d'oxydation choisie parmi les dérivés de paraphénylènediamine différents de la paraphénylènediamine , les bases doubles, les orthoaminophénols et les bases hétérocycliques,
- au moins une seconde base d'oxydation choisie parmi les para-aminophénols,
- au moins un méta-aminophénol à titre de coupleur,
- au moins une enzyme de type oxydo-réductase à 2 électrons,
- et au moins un donneur pour ladite enzyme.

2. Composition selon la revendication 1, **caractérisée par le fait que** l'oxydo-réductases à 2 électrons est choisie parmi les pyranose oxydases, les glucose oxydases, les glycérol oxydases, les lactates oxydases, les pyruvate oxydases, et les uricases.

3. Composition selon la revendication 1 ou 2, **caractérisée par le fait que** l'oxydo-réductases à 2 électrons est choisie parmi les uricases d'origine animale, microbiologique ou biotechnologique.

4. Composition selon l'une quelconque des revendications précédentes, **caractérisée par le fait que** la ou les oxydo-réductases à 2 électrons représentent de 0,01 à 20 % en poids du poids total de la composition tinctoriale prête à l'emploi.

5. Composition selon la revendication 4, **caractérisée par le fait que** la ou les oxydo-réductases à 2 électrons représentent de 0,1 à 5 % en poids du poids total de la composition tinctoriale prête à l'emploi.

6. Composition selon la revendication 3, **caractérisée par le fait que** le donneur (ou substrat) pour ladite oxydo-réductase à 2 électrons est choisi parmi l'acide urique et ses sels.

7. Composition selon l'une quelconque des revendications précédentes, **caractérisée par le fait que** le ou les donneurs représentent de 0,01 à 20 % en poids du poids total de la composition tinctoriale prête à l'emploi.

8. Composition selon la revendication 7, **caractérisée par le fait que** le ou les donneurs représentent de 0,1 à 5 % en poids du poids total de la composition tinctoriale prête à l'emploi.

9. Composition selon l'une quelconque des revendications précédentes, **caractérisée par le fait que** les para-aminophénols sont choisis parmi les composés répondant à la formule (II) suivante, et leurs sels d'addition avec un acide : 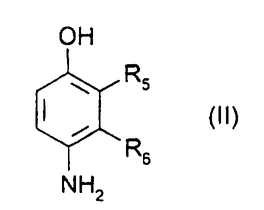 dans laquelle :
- R₅ représente un atome d'hydrogène ou d'halogène, un radical alkyle en C₁-C₄, monohydroxyalkyle en C₁-C₄, alcoxy(C₁-C₄)alkyle(C₁-C₄), aminoalkyle en C₁-C₄ ou hydroxyalkyl(C₁-C₄)aminoalkyle en C₁-C₄,
- R₆ représente un atome d'hydrogène ou d'halogène, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄),
étant entendu qu'au moins un des radicaux R₅ ou R₆ représente un atome d'hydrogène.

10. Composition selon la revendication 9, **caractérisée par le fait que** les para-aminophénols de formule (II) sont choisis parmi le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

11. Composition selon l'une quelconque des revendications précédentes, **caractérisée par le fait que** le ou les para-aminophénols représentent de 0,0005 à 12 % en poids du poids total de la composition tinctoriale prête à l'emploi.

12. Composition selon la revendication 11, **caractérisée par le fait que** le ou les para-aminophénols représentent de 0,005 à 6 % en poids du poids total de la composition tinctoriale prête à l'emploi.

13. Composition selon l'une quelconque des revendications précédentes, **caractérisée par le fait que** les méta-aminophénols sont choisis parmi les composés de formule (III) suivante, et leurs sels d'addition avec un acide : 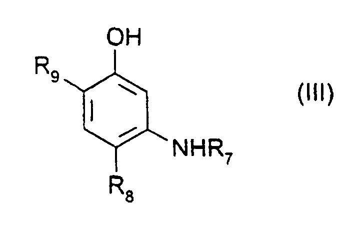 dans laquelle :
- R₇ représente un atome d'hydrogène, un radical alkyle en C₁-C₄, monohydroxyalkyle en C₁-C₄ ou polyhydroxyalkyle en C₂-C₄,
- R₈ représente un atome d'hydrogène, un radical alkyle en C₁-C₄, alcoxy en C₁-C₄ ou un atome d'halogène choisi parmi le chlore, le brome ou le fluor,
- R₉ représente un atome d'hydrogène, un radical alkyle en C₁-C₄, alcoxy en C₁-C₄, monohydroxyalkyle en C₁-C₄, polyhydroxyalkyle en C₂-C₄, monohydroxyalcoxy en C₁-C₄ ou polyhydroxyalcoxy en C₂-C₄.

14. Composition selon la revendication 13, **caractérisée par le fait que** les méta-aminophénol de formule (III) sont choisis parmi le méta-aminophénol, le 5-amino 2-méthoxy phénol, le 5-amino 2-(β-hydroxyéthyloxy) phénol, le 5-amino 2-méthyl phénol, le 5-N-(β-hydroxyéthyl)amino 2-méthyl phénol, le 5-N-(β-hydroxyéthyl)amino 4-méthoxy 2-méthyl phénol, le 5-amino 4-méthoxy 2-méthyl phénol, le 5-amino 4-chloro 2-méthyl phénol, le 5-amino 2,4-diméthoxy phénol, le 5-(γ-hydroxypropylamino) 2-méthyl phénol, et leurs sels d'addition avec un acide.

15. Composition selon l'une quelconque des revendications précédentes, **caractérisée par le fait que** le ou les méta-aminophénols représentent de 0,0001 à 8 % en poids du poids total de la composition tinctoriale prête à l'emploi.

16. Composition selon la revendication 15, **caractérisée par le fait que** le ou les méta-aminophénols représentent de 0,005 à 5 % en poids du poids total de la composition tinctoriale prête à l'emploi.

17. Composition selon l'une quelconque des revendications précédentes, **caractérisée par le fait que** les dérivés de paraphénylènediamine sont choisis parmi les composés de formule (I) suivante, et leurs sels d'addition avec un acide : 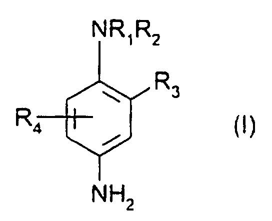 dans laquelle :
- R₁ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄), alkyle en C₁-C₄ substitué par un groupement azoté, phényle
ou 4'-aminophényle ;
- R₂ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄) ou alkyle en C₁-C₄ substitué par un groupement azoté ;
- R₃ représente un atome d'hydrogène, un atome d'halogène tel qu'un atome de chlore, de brome, d'iode ou de fluor, un radical alkyle en C₁-C₄, monohydroxyalkyle en C₁-C₄, hydroxyalcoxy en C₁-C₄, acétylaminoalcoxy en C₁-C₄, mésylaminoalcoxy en C₁-C₄ ou carbamoylaminoalcoxy en C₁-C₄,
- R₄ représente un atome d'hydrogène, d'halogène ou un radical alkyle en C₁-C₄ ;
étant entendu qu'au moins un des radicaux R₁ à R₄ est différent d'un atome d'hydrogène.

18. Composition selon la revendication 17, **caractérisée par le fait que** les dérivés de paraphénylènediamine de formule (I) sont choisis parmi la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxy-éthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphényiènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.

19. Composition selon l'une quelconque des revendications précédentes, **caractérisée par le fait que** les bases doubles sont choisies parmi les composés de formule (IV) suivante, et leurs sels d'addition avec un acide : 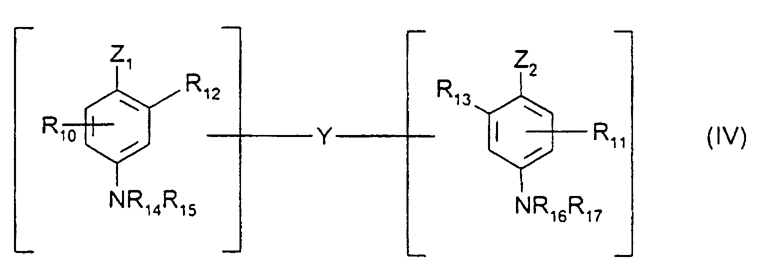 dans laquelle :
- Z₁ et Z₂, identiques ou différents, représentent un radical hydroxyle ou -NH₂ pouvant être substitué par un radical alkyle en C₁-C₄ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
- R₁₀ et R₁₁ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
- R₁₂, R₁₃, R₁₄, R₁₅, R₁₆ et R₁₇, identiques ou différents, représentent un atome d'hydrogène, un bras de liaison Y ou un radical alkyle en C₁-C₄ ;
étant entendu que les composés de formule (IV) ne comportent qu'un seul bras de liaison Y par molécule.

20. Composition selon la revendication 19, **caractérisée par le fait que** les bases doubles de formule (IV) sont choisies parmi le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

21. Composition selon l'une quelconque des revendications précédentes, **caractérisée par le fait que** les orthoaminophénols sont choisis parmi le 2-amino phénol, le 2-amino 5-méthyl phénol, le 2-amino 6-méthyl phénol, le 5-acétamido 2-amino phénol, et leurs sels d'addition avec un acide.

22. Composition selon l'une quelconque des revendications précédentes, **caractérisée par le fait que** les bases hétérocycliques sont choisies parmi les dérivés pyridiniques, les dérivés pyrimidiniques, les dérivés pyrazoliques, les dérivés pyrazolo-pyrimidiniques, et leurs sels d'addition avec un acide.

23. Composition selon l'une quelconque des revendications précédentes, **caractérisée par le fait que** le ou les dérivés de paraphénylènediamine et/ou la ou les bases doubles et/ou les ou les orthoaminophénols et/ou la ou les bases hétérocycliques représentent de 0,0005 à 12 % en poids du poids total de la composition tinctoriale prête à l'emploi.

24. Composition selon la revendication 23, **caractérisée par le fait que** le ou les dérivés de paraphénylènediamine et/ou la ou les bases doubles et/ou les ou les orthoaminophénols et/ou la ou les bases hétérocycliques représentent de 0,005 à 6 % en poids du poids total de la composition tinctoriale prête à l'emploi.

25. Composition selon l'une quelconque des revendications précédentes, **caractérisée par le fait que** les sels d'addition avec un acide sont choisis parmi les chlorhydrates, les bromhydrates, les sulfates et les tartrates, les lactates et les acétates.

26. Composition selon l'une quelconque des revendications précédentes, **caractérisée par le fait que** le milieu approprié pour la teinture est constitué par de l'eau ou par un mélange d'eau et d'au moins un solvant organique.

27. Composition selon l'une quelconque des revendications précédentes, **caractérisée par le fait qu'**elle présente un pH compris 5 et 11.

28. Composition selon l'une quelconque des revendications précédentes, **caractérisée par le fait qu'**elle contient au moins une peroxydase.

29. Procédé de teinture des fibres kératiniques et en particulier des fibres kératiniques humaines telles que les cheveux, **caractérisé par le fait qu'**on applique sur lesdites fibres au moins une composition tinctoriale prête à l'emploi telle que définie dans l'une quelconque des revendications précédentes, pendant un temps suffisant pour développer la coloration désirée.

30. Procédé selon la revendication 29, **caractérisé par le fait qu'**il comporte une étape préliminaire consistant à stocker sous forme séparée, d'une part, une composition (A) comprenant, dans un milieu approprié pour la teinture au moins une première base d'oxydation choisie parmi les dérivés de paraphénylènediamine, les bases doubles, les orthoaminophénols et les bases hétérocycliques, au moins une seconde base d'oxydation choisie parmi les para-aminophénols, au moins un méta-aminophénol à titre de coupleur et, d'autre part, une composition (B) renfermant, dans un milieu approprié pour la teinture, au moins une enzyme de type oxydo-réductase à 2 électrons en présence d'au moins un donneur pour ladite enzyme, puis à procéder à leur mélange au moment de l'emploi avant d'appliquer ce mélange sur les fibres kératiniques.

31. Dispositif à plusieurs compartiments ou "kit" de teinture, **caractérisé par le fait qu'**il comporte un premier compartiment renfermant la composition (A) telle que définie dans la revendication 30 et un second compartiment renfermant la composition (B) telle que définie dans la revendication 30.

## Patentansprüche

1. Gebrauchsfertige Zusammensetzung zum oxidativen Färben von Keratinfasern und insbesondere menschlichen Keratinfasern, wie dem Haar, die in einem zum Färben geeigneten Träger enthält:
· mindestens eine erste Oxidationsbase, die unter den p-Phenylendiaminderivaten, die von p-Phenylendiamin verschieden sind, Doppelbasen, o-Aminophenolen und heterocyclischen Basen ausgewählt ist,
· mindestens eine zweite Oxidationsbase, die unter den p-Aminophenolen ausgewählt ist,
· mindestens ein m-Aminophenol als Kuppler,
· mindestens ein Enzym vom Typ der Oxidoreduktasen (2 Elektronen), und
· mindestens einen Donor für das Enzym.

2. Zusammensetzung nach Anspruch 1, **dadurch gekennzeichnet, dass** die Oxidoreduktasen (2 Elektronen) unter den Pyranoseoxidasen, Glucoseoxidasen, Glycerinoxidasen, Lactatoxidasen, Pyruvatoxidasen und Uricasen ausgewählt sind.

3. Zusammensetzung nach Anspruch 1 oder 2, **dadurch gekennzeichnet, dass** die Oxidoreduktasen (2 Elektronen) unter den Uricasen tierischer, mikrobiologischer oder biotechnologischer Herkunft ausgewählt sind.

4. Zusammensetzung nach einem der vorhergehenden Ansprüche, **dadurch gekennzeichnet, dass** die Oxidoreduktase(n) (2 Elektronen) 0,01 bis 20 Gew.-% des Gesamtgewichts der gebrauchsfertigen Farbmittelzusammensetzung ausmachen.

5. Zusammensetzung nach Anspruch 4, **dadurch gekennzeichnet, dass** die Oxidoreduktase(n) (2 Elektronen) 0,1 bis 5 Gew.-% des Gesamtgewichts der gebrauchsfertigen Farbmittelzusammensetzung ausmachen.

6. Zusammensetzung nach Anspruch 3, **dadurch gekennzeichnet, dass** der Donor (oder das Substrat) für die Oxidoreduktasen (2 Elektronen) unter Harnsäure und ihren Salzen ausgewählt ist.

7. Zusammensetzung nach einem der vorhergehenden Ansprüche, **dadurch gekennzeichnet, dass** der Donor oder die Donoren 0,01 bis 20 Gew.-% des Gesamtgewichts der gebrauchsfertigen Farbmittelzusammensetzung ausmachen.

8. Zusammensetzung nach Anspruch 7, **dadurch gekennzeichnet, dass** der Donor oder die Donoren 0,1 bis 5 Gew.-% des Gesamtgewichts der gebrauchsfertigen Farbmittelzusammensetzung ausmachen.

9. Zusammensetzung nach einem der vorhergehenden Ansprüche, **dadurch gekennzeichnet, dass** die p-Aminophenole unter den Verbindungen der folgenden Formel (II) und deren Additionssalze mit einer Säure ausgewählt sind: 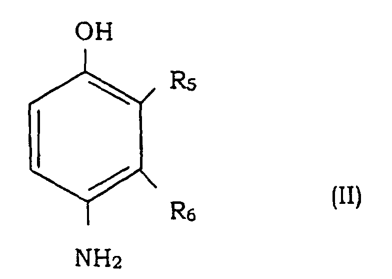 worin bedeuten:
· R₅ Wasserstoff, Halogen, C₁₋₄-Alkyl, C₁₋₄-Monohydroxyalkyl, C₁₋₄-Alkoxy-C₁₋₄-alkyl, C₁₋₄-Aminoalkyl oder C₁₋₄-Hydroxyalkyl-C₁₋₄-aminoalkyl,
· R₆ Wasserstoff, Halogen, C₁₋₄-Alkyl, C₁₋₄-Monohydroxyalkyl, C₂₋₄-Polyhydroxyalkyl, C₁₋₄-Aminoalkyl, C₁₋₄-Cyanoalkyl oder C₁₋₄-Alkoxy-C₁₋₄-alkyl,
mit der Maßgabe, dass mindestens eine der Gruppen R₅ oder R₆ Wasserstoff bedeutet.

10. Zusammensetzung nach Anspruch 9, **dadurch gekennzeichnet, dass** die p-Aminophenole der Formel (II) unter p-Aminophenol, 4-Amino-3-methyl-phenol, 4-Amino-3-fluor-phenol, 4-Amino-3-hydroxymethyl-phenol, 4-Amino-2-methyl-phenol, 4-Amino-2-hydroxymethyl-phenol, 4-Amino-2-methoxymethyl-phenol, 4-Amino-2-aminomethyl-phenol, 4-Amino-2-(β-hydroxyethylaminomethyl)-phenol, 4-Amino-2-fluor-phenol und deren Additionssalze mit einer Säure ausgewählt sind.

11. Zusammensetzung nach einem der vorhergehenden Ansprüche, **dadurch gekennzeichnet, dass** das p-Aminophenol oder die p-Aminophenole 0,0005 bis 12 Gew.-% des Gesamtgewichts der erfindungsgemäßen gebrauchsfertigen Farbmittelzusammensetzung ausmachen.

12. Zusammensetzung nach Anspruch 11, **dadurch gekennzeichnet, dass** das p-Aminophenol oder die p-Aminophenole 0,005 bis 6 Gew.-% des Gesamtgewichts der erfindungsgemäßen gebrauchsfertigen Farbmittelzusammensetzung ausmachen.

13. Zusammensetzung nach einem der vorhergehenden Ansprüche, **dadurch gekennzeichnet, dass** die m-Aminophenole unter den Verbindungen der folgenden Formel (III) und deren Additionssalzen mit einer Säure ausgewählt sind: 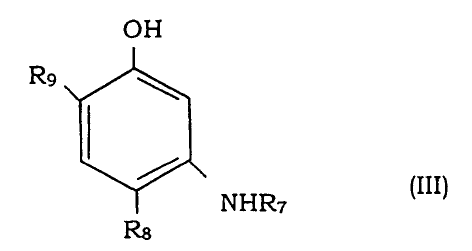 worin bedeuten:
· R₇ Wasserstoff, C₁₋₄-Alkyl, C₁₋₄-Monohydroxyalkyl oder C₂₋₄-Polyhydroxyalkyl;
· R₈ Wasserstoff, C₁₋₄-Alkyl, C₁₋₄-Alkoxy oder ein Halogenatom, das unter Chlor, Brom oder Fluor ausgewählt ist,
· R₉ Wasserstoff, C₁₋₄-Alkyl, C₁₋₄-Alkoxy, C₁₋₄-Monohydroxyalkyl, C₂₋₄-Polyhydroxyalkyl, C₁₋₄-Monohydroxyalkoxy oder C₂₋₄-Polyhydroxyalkoxy.

14. Zusammensetzung nach Anspruch 13, **dadurch gekennzeichnet, dass** die m-Aminophenole der Formel (III) unter m-Aminophenol, 5-Amino-2-methoxy-phenol, 5-Amino-2-(β-hydroxyethyloxy)-phenol, 5-Amino-2-methyl-phenol, 5-N-(β-Hydroxyethyl)amino-2-methyl-phenol, 5-N-(β-Hydroxyethyl)amino-4-methoxy-2-methylphenol, 5-Amino-4-methoxy-2-methyl-phenol, 5-Amino-4-chlor-2-methyl-phenol, 5-Amino-2,4-dimethoxy-phenol, 5-(γ-Hydroxypropylamino)-2-methyl-phenol und den Additionssalzen dieser Verbindungen mit einer Säure ausgewählt sind.

15. Zusammensetzung nach einem der vorhergehenden Ansprüche, **dadurch gekennzeichnet, dass** das m-Aminophenol oder die m-Aminophenole 0,0001 bis 8 Gew.-% des Gesamtgewichts der erfindungsgemäßen gebrauchsfertigen Farbmittelzusammensetzung ausmachen.

16. Zusammensetzung nach Anspruch 15, **dadurch gekennzeichnet, dass** das m-Aminophenol oder die m-Aminophenole 0,005 bis 5 Gew.-% des Gesamtgewichts der erfindungsgemäßen gebrauchsfertigen Farbmittelzusammensetzung ausmachen.

17. Zusammensetzung nach einem der vorhergehenden Ansprüche, **dadurch gekennzeichnet, dass** die p-Phenylendiaminderivate unter den Verbindungen der folgenden Formel (I) und deren Additionssalzen mit einer Säure ausgewählt sind: 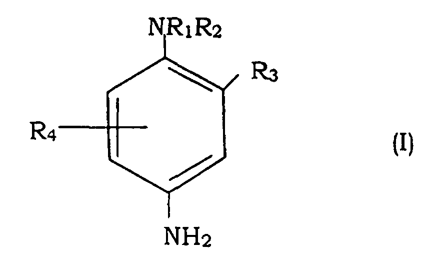 worin bedeuten:
· R₁ Wasserstoff, C₁₋₄-Alkyl, C₁₋₄-Monohydroxyalkyl, C₂₋₄-Polyhydroxyalkyl, C₁₋₄-Alkoxy-C₁₋₄-alkyl, eine mit einer stickstoffhaltigen Gruppe substituierte C₁₋₄-Alkylgruppe, Phenyl oder 4'-Aminophenyl,
· R₂ Wasserstoff, C₁₋₄-Alkyl, C₁₋₄-Monohydroxyalkyl, C₂₋₄-Polyhydroxyalkyl, C₁₋₄-Alkoxy-C₁₋₄-alkyl oder eine mit einer stickstoffhaltigen Gruppe substituierte C₁₋₄-Alkylgruppe,
· R₃ Wasserstoff, Halogen, wie Chlor, Brom, Iod oder Fluor, C₁₋₄-Alkyl, C₁₋₄-Monohydroxyalkyl, C₁₋₄-Hydroxyalkoxy, C₁₋₄-Acetylaminoalkoxy, C₁₋₄-Mesylaminoalkoxy oder C₁₋₄-Carbamoylaminoalkoxy, und
· R₄ Wasserstoff, Halogen oder C₁₋₄-Alkyl,
mit der Maßgabe, dass mindestens eine der Gruppen R₁ bis R₄ von Wasserstoff verschieden ist.

18. Zusammensetzung nach Anspruch 17, **dadurch gekennzeichnet, dass** die p-Phenylendiamine der Formel (I) unter p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-pphenylendiamin, N,N-Diethyl-p-phenylendiamin, N,N-Dipropyl-pphenylendiamin, 4-Amino-N,N-diethyl-3-methyl-anilin, N,N-Bis(β-hydroxyethyl)-p-phenylendiamin, 4-N,N-Bis(β-hydroxyethyl)-amino-2-methyl-anilin, 4-N,N-Bis(β-hydroxyethyl)amino-2-chloranilin, 2-β-Hydroxyethyl-p-phenylendiamin, 2-Fluor-p-phenylendiamin, 2-Isopropyl-p-phenylendiamin, N-(β-Hydroxypropyl)-pphenylendiamin, 2-Hydroxymethyl-p-phenylendiamin, N,N-Dimethyl-3-methyl-p-phenylendiamin, N,N-(Ethyl, β-hydroxyethyl)-p-phenylendiamin, N-(β,γ-Dihydroxypropyl)-p-phenylendiamin, N-(4'-Aminophenyl)-p-phenylendiamin, N-Phenyl-pphenylendiamin, 2-β-Hydroxyethyloxy-p-phenylendiamin, 2-β-Acetylaminoethyloxy-p-phenylendiamin, N-(β-Methoxyethyl)-pphenylendiamin und deren Additionssalzen mit einer Säure ausgewählt sind.

19. Zusammensetzung nach einem der vorhergehenden Ansprüche, **dadurch gekennzeichnet, dass** die Doppelbasen unter den Verbindungen der folgenden Formel (IV) und deren Additionssalze mit einer Säure ausgewählt sind: 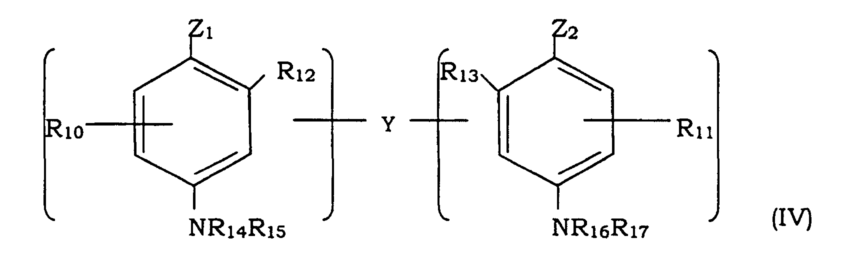 worin bedeuten:
· Z₁ und Z₂, die identisch oder voneinander verschieden sind, eine Hydroxygruppe oder eine NH₂-Gruppe, die mit C₁₋₄-Alkyl oder einer Verbindungsgruppe Y substituiert sein kann,
· die Verbindungsgruppe Y eine geradkettige oder verzweigte Alkylengruppe mit 1 bis 14 Kohlenstoffatomen, die durch eine oder mehrere stickstoffhaltige Gruppen und/ oder ein oder mehrere Heteroatome, wie Sauerstoff, Schwefel oder Stickstoff, unterbrochen oder abgeschlossen werden kann und gegebenenfalls mit einer oder mehreren Hydroxy- oder C₁₋₆-Alkoxygruppen substituiert sein kann,
· R₁₀ und R₁₁ Wasserstoff, Halogen, C₁₋₄-Alkyl, C₁₋₄-Monohydroxyalkyl, C₂₋₄-Polyhydroxyalkyl, C₁₋₄-Aminoalkyl oder eine Verbindungsgruppe Y,
· R₁₂, R₁₃, R₁₄, R₁₅, R₁₆ und R₁₇, die gleich oder verschieden sind, Wasserstoff, eine Verbindungsgruppe Y oder C₁₋₄-Alkyl,
mit der Maßgabe, dass die Verbindungen der Formel (IV) nur eine Verbindungsgruppe Y pro Molekül aufweisen.

20. Zusammensetzung nach Anspruch 19, **dadurch gekennzeichnet, dass** die Doppelbasen der Formel (IV) unter N,N'-Bis(β-hydroxyethyl)-N,N'-bis-(4'-aminophenyl)-1,3-diaminopropanol, N,N'-Bis(β-hydroxyethyl)-N,N'-bis(4'-aminophenyl)-ethylendiamin, N,N'-Bis(4-aminophenyl)-tetramethylendiamin, N,N'-Bis(β-hydroxyethyl)-N,N'-bis(4-aminophenyl)-tetramethylendiamin, N,N'-Bis(4-methylaminophenyl)-tetramethylendiamin, N,N'-Bis(ethyl)-N,N'bis(4'-amino-3'-methylphenyl)-ethylendiamin, 1,8-Bis(2,5-diaminophenoxy)-3,5-dioxaoctan und deren Additionssalzen mit einer Säure ausgewählt sind.

21. Zusammensetzung nach einem der vorhergehenden Ansprüche, **dadurch gekennzeichnet, dass** die o-Aminophenole unter 2-Aminophenol, 2-Amino-5-methyl-phenol, 2-Amino-6-methylphenol, 5-Acetamido-2-amino-phenol und deren Additionssalzen mit einer Säure ausgewählt sind.

22. Zusammensetzung nach einem der vorhergehenden Ansprüche, **dadurch gekennzeichnet, dass** die heterocyclischen Oxidationsbasen unter den Pyridinderivaten, Pyrimidinderivaten, Pyrazolderivaten, Pyrazolo-pyrimidinderivaten und deren Additionssalzen mit einer Säure ausgewählt sind.

23. Zusammensetzung nach einem der vorhergehenden Ansprüche, **dadurch gekennzeichnet, dass** das oder die p-Phenylendiaminderivat(e) und/oder die Doppelbase(n) und/oder das oder die o-Aminophenol(e) und/oder die heterocyclische(n) Base(n) 0,0005 bis 12 Gew.-% des Gesamtgewichts der erfindungsgemäßen gebrauchsfertigen Farbmittelzusammensetzung ausmachen.

24. Zusammensetzung nach Anspruch 23, **dadurch gekennzeichnet, dass** das oder die p-Phenylendiaminderivat(e) und/oder die Doppelbase(n) und/oder das oder die o-Aminophenol(e) und/oder die heterocyclische(n) Base(n) 0,005 bis 6 Gew.-% des Gesamtgewichts der erfindungsgemäßen gebrauchsfertigen Farbmittelzusammensetzung ausmachen.

25. Zusammensetzung nach einem der vorhergehenden Ansprüche, **dadurch gekennzeichnet, dass** die Additionssalze mit einer Säure unter den Hydrochloriden, Hydrobromiden, Sulfaten, Tartraten, Lactaten und Acetaten ausgewählt sind.

26. Zusammensetzung nach einem der vorhergehenden Ansprüche, **dadurch gekennzeichnet, dass** das zum Färben geeignete Medium aus Wasser oder einem Gemisch von Wasser und mindestens einem organischen Lösungsmittel besteht.

27. Zusammensetzung nach einem der vorhergehenden Ansprüche, **dadurch gekennzeichnet, dass** sie einen pH-Wert im Bereich von 5 bis 11 aufweist.

28. Zusammensetzung nach einem der vorhergehenden Ansprüche, **dadurch gekennzeichnet, dass** sie mindestens eine Peroxidase enthält.

29. Verfahren zum Färben von Keratinfasern und insbesondere menschlichen Keratinfasern, wie dem Haar, **dadurch gekennzeichnet, dass** auf die Fasern mindestens eine gebrauchsfertige Farbmittelzusammensetzung nach einem der vorhergehenden Ansprüche während einer Zeitspanne, die zur Entwicklung der gewünschten Färbung ausreichend ist, aufgebracht wird.

30. Verfahren nach Anspruch 29, **dadurch gekennzeichnet, dass** es einen vorbereitenden Schritt umfasst, der darin besteht, einerseits eine Zusammensetzung (A), die in einem zum Färben geeigneten mindestens eine erste Oxidationsbase, die unter den p-Phenylendiaminderivaten, Doppelbasen, o-Aminophenolen und heterocyclischen Basen ausgewählt ist, mindestens eine zweite Oxidationsbase, die unter den p-Aminophenolen ausgewählt ist, und mindestens ein m-Aminophenol als Kuppler enthält, und andererseits eine Zusammensetzung (B) getrennt voneinander aufzubewahren, die in einem zum Färben geeigneten Medium mindestens ein Enzym vom Typ Oxidoreduktase (2 Elektronen) in Gegenwart mindestens eines Donors für das Enzym enthält, und diese bei der Anwendung zu vermischen, bevor das Gemisch auf die Fasern aufgebracht wird.

31. Vorrichtung mit mehreren Abteilungen oder "Kit" zum Färben, **dadurch gekennzeichnet, dass** sie eine erste Abteilung mit der in Anspruch 30 definierten Zusammensetzung (A) und eine zweite Abteilung mit der in Anspruch 30 definierten Zusammensetzung (B) enthält.

## Claims

1. Ready-to-use composition for the oxidation dyeing of keratin fibres, and in particular human keratin fibres such as the hair, **characterized in that** it comprises, in a medium which is suitable for dyeing:
- at least one first oxidation base chosen from para-phenylenediamine derivatives other than para-phenylenediamine, double bases, ortho-aminophenols and heterocyclic bases,
- at least one second oxidation base chosen from para-aminophenols,
- at least one meta-aminophenol as coupler,
- at least one enzyme of 2-electron oxidoreductase type, and
- at least one donor for the said enzyme.

2. Composition according to Claim 1, **characterized in that** the 2-electron oxidoreductase is chosen from pyranose oxidases, glucose oxidases, glycerol oxidases, lactate oxidases, pyruvate oxidases and uricases.

3. Composition according to Claim 1 or 2, **characterized in that** the 2-electron oxidoreductase is chosen from uricases of animal, microbiological or biotechnological origin.

4. Composition according to any one of the preceding claims, **characterized in that** the 2-electron oxidoreductase(s) represent(s) from 0.01 to 20% by weight relative to the total weight of the ready-to-use dye composition.

5. Composition according to Claim 4, **characterized in that** the 2-electron oxidoreductase(s) represent(s) from 0.1 to 5% by weight relative to the total weight of the ready-to-use dye composition.

6. Composition according to Claim 3, **characterized in that** the donor (or substrate) for the said 2-electron oxidoreductase is chosen from uric acid and its salts.

7. Composition according to any one of the preceding claims, **characterized in that** the donor(s) represent(s) from 0.01 to 20% by weight relative to the total weight of the ready-to-use dye composition.

8. Composition according to Claim 7, **characterized in that** the donor(s) represent(s) from 0.1 to 5% by weight relative to the total weight of the ready-to-use dye composition.

9. Composition according to any one of the preceding claims, **characterized in that** the para-aminophenols are chosen from the compounds corresponding to formula (II) below, and the addition salts thereof with an acid: 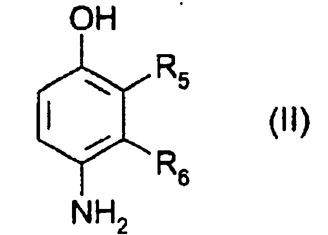 in which:
- R₅ represents a hydrogen or halogen atom or a C₁-C₄ alkyl, C₁-C₄ monohydroxyalkyl, (C₁-C₄)alkoxy(C₁-C₄)alkyl, C₁-C₄ aminoalkyl or hydroxy(C₁-C₄)alkylamino(C₁-C₄)alkyl radical,
- R₆ represents a hydrogen or halogen atom or a C₁-C₄ alkyl, C₁-C₄ monohydroxyalkyl, C₂-C₄ polyhydroxyalkyl, C₁-C₄ aminoalkyl, cyano(C₁-C₄)alkyl or (C₁-C₄)alkoxy(C₁-C₄)alkyl radical,
it being understood that at least one of the radicals R₅ or R₆ represents a hydrogen atom.

10. Composition according to Claim 9, **characterized in that** the para-aminophenols of formula (II) are chosen from para-aminophenol, 4-amino-3-methylphenol, 4-amino-3-fluorophenol, 4-amino-3-hydroxymethylphenol, 4-amino-2-methylphenol, 4-amino-2-hydroxymethylphenol, 4-amino-2-methoxymethylphenol, 4-amino-2-aminomethylphenol, 4-amino-2-(β-hydroxyethylaminomethyl)phenol and 4-amino-2-fluorophenol, and the addition salts thereof with an acid.

11. Composition according to any one of the preceding claims, **characterized in that** the para-aminophenol(s) represent(s) from 0.0005 to 12% by weight relative to the total weight of the ready-to-use dye composition.

12. Composition according to Claim 11, **characterized in that** the para-aminophenol(s) represent(s) from 0.005 to 6% by weight relative to the total weight of the ready-to-use dye composition.

13. Composition according to any one of the preceding claims, **characterized in that** the meta-aminophenols are chosen from the compounds of formula (III) below, and the addition salts thereof with an acid: 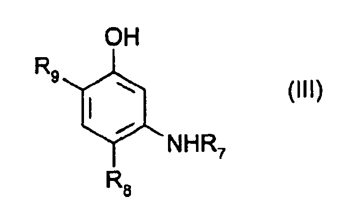 in which:
- R₇ represents a hydrogen atom or a C₁-C₄ alkyl, C₁-C₄ monohydroxyalkyl or C₂-C₄ polyhydroxyalkyl radical,
- R₈ represents a hydrogen atom, a C₁-C₄ alkyl or C₁-C₄ alkoxy radical or a halogen atom chosen from chlorine, bromine and fluorine,
- R₉ represents a hydrogen atom or a C₁-C₄ alkyl, C₁-C₄ alkoxy, C₁-C₄ monohydroxyalkyl, C₂-C₄ polyhydroxyalkyl, C₁-C₄ monohydroxyalkoxy or C₂-C₄ polyhydroxyalkoxy radical.

14. Composition according to Claim 13, **characterized in that** the meta-aminophenols of formula (III) are chosen from meta-aminophenol, 5-amino-2-methoxyphenol, 5-amino-2-(β-hydroxyethyloxy)phenol, 5-amino-2-methylphenol, 5-N-(β-hydroxyethyl)amino-2-methylphenol, 5-N-(β-hydroxyethyl)amino-4-methoxy-2-methylphenol, 5-amino-4-methoxy-2-methylphenol, 5-amino-4-chloro-2-methylphenol, 5-amino-2,4-dimethoxyphenol and 5-(γ-hydroxypropylamino)-2-methylphenol and the addition salts thereof with an acid.

15. Composition according to any one of the preceding claims, **characterized in that** the meta-aminophenol(s) represent(s) from 0.0001 to 8% by weight relative to the total weight of the ready-to-use dye composition.

16. Composition according to Claim 15, **characterized in that** the meta-aminophenol(s) represent(s) from 0.005 to 5% by weight relative to the total weight of the ready-to-use dye composition.

17. Composition according to any one of the preceding claims, **characterized in that** the para-phenylenediamine derivatives are chosen from the compounds of formula (I) below, and the addition salts thereof with an acid: 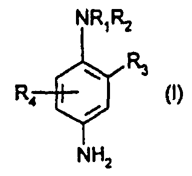 in which:
- R₁ represents a hydrogen atom, a C₁-C₄ alkyl radical, a C₁-C₄ monohydroxyalkyl radical, a C₂-C₄ polyhydroxyalkyl radical, a (C₁-C₄)alkoxy(C₁-C₄)alkyl radical, a C₁-C₄ alkyl radical substituted with a nitrogenous group, a phenyl radical or a 4'-aminophenyl radical;
- R₂ represents a hydrogen atom, a C₁-C₄ alkyl radical, a C₁-C₄ monohydroxyalkyl radical, a C₂-C₄ polyhydroxyalkyl radical, a (C₁-C₄)alkoxy(C₁-C₄)alkyl radical or a C₁-C₄ alkyl radical substituted with a nitrogenous group;
- R₃ represents a hydrogen atom, a halogen atom such as a chlorine, bromine, iodine or fluorine atom, a C₁-C₄ alkyl radical, a C₁-C₄ monohydroxyalkyl radical, a C₁-C₄ hydroxyalkoxy radical, an acetylamino(C₁-C₄)-alkoxy radical, a C₁-C₄ mesylaminoalkoxy radical or a carbamoylamino (C₁-C₄) alkoxy radical,
- R₄ represents a hydrogen or halogen atom or a C₁-C₄ alkyl radical;
it being understood that at least one of the radicals R₁ to R₄ is other than a hydrogen atom.

18. Composition according to Claim 17, **characterized in that** the para-phenylenediamine derivatives of formula (I) are chosen from para-tolylenediamine, 2-chloro-para-phenylenediamine, 2,3-dimethyl-para-phenylenediamine, 2,6-dimethyl-para-phenylenediamine, 2,6-diethyl-para-phenylenediamine, 2,5-dimethyl-para-phenylenediamine, N,N-dimethyl-para-phenylenediamine, N,N-diethyl-para-phenylenediamine, N,N-dipropyl-para-phenylenediamine, 4-amino-N,N-diethyl-3-methylaniline, N,N-bis(β-hydroxyethyl)-para-phenylenediamine, 4-amino-N,N-bis(β-hydroxyethyl)-2-methylaniline, 4-amino-2-chloro-N,N-bis(β-hydroxyethyl) aniline, 2-β-hydroxyethyl-para-phenylenediamine, 2-fluoro-para-phenylenediamine, 2-isopropyl-para-phenylenediamine, N-(β-hydroxypropyl)-para-phenylenediamine, 2-hydroxymethyl-para-phenylenediamine, N,N-dimethyl-3-methyl-para-phenylenediamine, N,N-(ethyl-β-hydroxyethyl)-para-phenylenediamine, N-(β,γ-dihydroxypropyl)-para-phenylenediamine, N-(4'-aminophenyl)-para-phenylenediamine, N-phenyl-para-phenylenediamine, 2-β-hydroxyethyloxy-para-phenylenediamine, 2-β-acetylaminoethyloxy-para-phenylenediamine and N-(β-methoxyethyl)-para-phenylenediamine, and the addition salts thereof with an acid.

19. Composition according to any one of the preceding claims, **characterized in that** the double bases are chosen from the compounds of formula (IV) below, and the addition salts thereof with an acid: 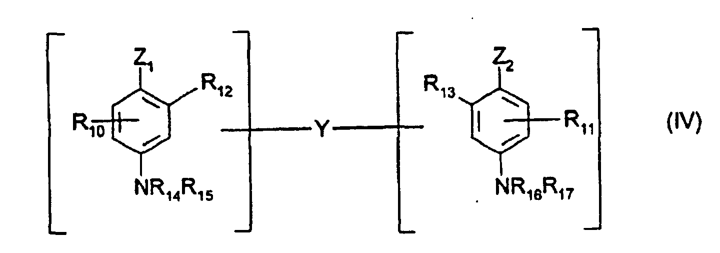 in which:
- Z₁ and Z₂, which may be identical or different, represent a hydroxyl or -NH₂ radical which may be substituted with a C₁-C₄ alkyl radical or with a linker arm Y;
- the linker arm Y represents a linear or branched alkylene chain containing from 1 to 14 carbon atoms, which may be interrupted by or terminated with one or more nitrogenous groups and/or one or more hetero atoms such as oxygen, sulphur or nitrogen atoms, and optionally substituted with one or more hydroxyl or C₁-C₆ alkoxy radicals;
- R₁₀ and R₁₁ represent a hydrogen or halogen atom, a C₁-C₄ alkyl radical, a C₁-C₄ monohydroxyalkyl radical, a C₂-C₄ polyhydroxyalkyl radical, a C₁-C₄ aminoalkyl radical or a linker arm Y;
- R₁₂, R₁₃, R₁₄, R₁₅, R₁₆ and R₁₇, which may be identical or different, represent a hydrogen atom, a linker arm Y or a C₁-C₄ alkyl radical;
it being understood that the compounds of formula (IV) contain only one linker arm Y per molecule.

20. Composition according to Claim 19, **characterized in that** the double bases of formula (IV) are chosen from N,N'-bis(β-hydroxyethyl)-N,N'-bis(4'-aminophenyl)-1,3-diaminopropanol, N,N'-bis(β-hydroxyethyl)-N,N'-bis(4'-aminophenyl)ethylenediamine, N,N'-bis(4-aminophenyl)tetramethylenediamine, N,N'-bis(β-hydroxyethyl)-N,N'-bis(4-aminophenyl)-tetramethylenediamine, N,N'-bis(4-methylaminophenyl)-tetramethylenediamine, N,N'-bis(ethyl)-N,N'-bis(4'-amino-3'-methylphenyl)ethylenediamine and 1,8-bis(2,5-diaminophenoxy)-3,5-dioxaoctane, and the addition salts thereof with an acid.

21. Composition according to any one of the preceding claims, **characterized in that** the ortho-aminophenols are chosen from 2-aminophenol, 2-amino-5-methylphenol, 2-amino-6-methylphenol and 5-acetamido-2-aminophenol, and the addition salts thereof with an acid.

22. Composition according to any one of the preceding claims, **characterized in that** the heterocyclic bases are chosen from pyridine derivatives, pyrimidine derivatives, pyrazole derivatives and pyrazolopyrimidine derivatives, and the addition salts thereof with an acid.

23. Composition according to any one of the preceding claims, **characterized in that** the para-phenylenediamine derivative(s) and/or the double base(s) and/or the ortho-aminophenol(s) and/or the heterocyclic base(s) represent(s) from 0.0005 to 12% by weight relative to the total weight of the ready-to-use dye composition.

24. Composition according to Claim 23, **characterized in that** the para-phenylenediamine derivative(s) and/or the double base(s) and/or the ortho-aminophenol(s) and/or the heterocyclic base(s) represent(s) from 0.005 to 6% by weight relative to the total weight of the ready-to-use dye composition.

25. Composition according to any one of the preceding claims, **characterized in that** the addition salts with an acid are chosen from the hydrochlorides, hydrobromides, sulphates, tartrates, lactates and acetates.

26. Composition according to any one of the preceding claims, **characterized in that** the medium which is suitable for dyeing consists of water or a mixture of water and at least one organic solvent.

27. Composition according to any one of the preceding claims, **characterized in that** it has a pH of between 5 and 11.

28. Composition according to any one of the preceding claims, **characterized in that** it contains at least one peroxidase.

29. Process for dyeing keratin fibres, and in particular human keratin fibres such as the hair, **characterized in that** at least one ready-to-use dye composition as defined in any one of the preceding claims is applied to the said fibres, for a period which is sufficient to develop the desired coloration.

30. Process according to Claim 29, **characterized in that** it includes a preliminary step which consists in separately storing, on the one hand, a composition (A) comprising, in a medium which is suitable for dyeing, at least one first oxidation base chosen from para-phenylenediamine derivatives, double bases, ortho-aminophenols and heterocyclic bases, at least one second oxidation base chosen from para-aminophenols, at least one meta-aminophenol as coupler, and, on the other hand, a composition (B) comprising, in a medium which is suitable for dyeing, at least one enzyme of 2-electron oxidoreductase type in the presence of at least one donor for the said enzyme, and then in mixing them together at the time of use, after which this mixture is applied to the keratin fibres.

31. Multi-compartment dyeing device or kit, **characterized in that** it includes a first compartment comprising composition (A) as defined in Claim 30 and a second compartment comprising composition (B) as defined in Claim 30.
